(19) Europäisches Patentamt
European Patent Office
Office européen des brevets

(11) Veröffentlichungsnummer: **0 139 252 B1**

# (12) EUROPÄISCHE PATENTSCHRIFT

(45) Veröffentlichungstag der Patentschrift: **08.11.89**

(51) Int. Cl.⁴: **C 07 C 67/03**, C 07 C 67/08 // C07C69/614, C07C69/612, C07C69/78

(21) Anmeldenummer: **84111517.3**

(22) Anmeldetag: **27.09.84**

(54) **Veresterung phenolgruppenhaltiger Carbonsäuren.**

(30) Priorität: **05.10.83 DE 3336199**

(43) Veröffentlichungstag der Anmeldung: **02.05.85 Patentblatt 85/18**

(45) Bekanntmachung des Hinweises auf die Patenterteilung: **08.11.89 Patentblatt 89/45**

(84) Benannte Vertragsstaaten: **AT BE CH DE FR GB IT LI NL**

(56) Entgegenhaltungen:
**DE-A- 2 247 309**
**DE-A- 3 242 307**

(73) Patentinhaber: **HOECHST AKTIENGESELLSCHAFT, Postfach 80 03 20, D-6230 Frankfurt am Main 80 (DE)**

(72) Erfinder: **Nestler, Hans Jürgen, Dr., Steinweg 15, D-6240 Königstein/Taunus (DE)**

EP 0 139 252 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Während die selektive Alkylierung einer aliphatischen oder aromatischen Carbonsäure zu ihrem Ester auch bei Anwesenheit alkoholischer Hydroxygruppen im selben Molekül meist problemlos gelingt, stellt diese Reaktion bei Carbonsäuren, die phenolische Hydroxygruppen im selben Molekül enthalten, eine anspruchsvolle technische Aufgabe dar. Wegen der in vergleichbarer Größenordnung liegenden Aciditäten von Carboxyl- und Phenolfunktion – bzw. der Nucleophilie von Carboxylat und Phenolat – tritt gleichzeitig zur Veresterung der Carbonsäuren auch eine Verätherung der phenolischen Gruppe in teilweise beträchtlichem Umfang ein. Dieses ist insbesondere der Fall, wenn man, um eine möglichst vollständige Veresterung zu erreichen, sogenannte «starke» Alkylierungsmittel verwendet wie z. B. Dialkylsulfate, Trialkyloxoniumsalze, Diazoalkane, Alkyl- und Arylsulfonsäureester oder auch Alkylhalogenide in Gegenwart von Silberionen.

Bei Anwendung milderer Alkylierungsbedingungen wie z. B. bei Umsetzung der Carbonsäuren mit den betreffenden Alkoholen in Gegenwart katalytischer Mengen von Mineralsäuren, aromatischer Sulfonsäuren oder Ionenaustauscherharzen bleiben zwar die phenolischen Gruppen unverändert erhalten, die Veresterungsreaktion führt aber bekanntlich nur bis zum Gleichgewichtsumsatz; der vollständige Ablauf der Reaktion wird durch das entstandene Reaktionswasser verhindert.

Der üblicherweise gewählte Ausweg, das Reaktionswasser beispielsweise durch azeotrope Destillation oder mit Hilfe von Schleppmitteln aus dem Veresterungsgemisch zu entfernen, versagt meist bei den technisch interessanten niedrigsiedenden Alkoholen Methanol, Ethanol, n-Propanol und Isopropanol. Eine weitere Komplikation ergibt sich dadurch, daß bei dem Versuch, das Wasser möglichst vollständig zu entfernen, die Phenoletherbildung als Nebenreaktion wieder deutlich in Erscheinung tritt. Außerdem findet unter diesen Bedingungen oft zusätzlich eine Polykondensation der phenolgruppenhaltigen Carbonsäuren statt, die zu unerwünschten hochmolekularen Nebenprodukten führt.

Die präparative Aufarbeitung solcher unvollständig oder unter Bildung von Nebenprodukten abgelaufenen Veresterungsansätze erweist sich aus folgenden Gründen als schwierig:

1. Die gebräuchliche alkalische Extraktion zur Entfernung nicht umgesetzter Säure ist wegen der gleichzeitig vorhandenen Phenolgruppe nicht anwendbar.

2. Jede wäßrige Aufarbeitung wird dadurch erschwert, daß die Ausgangs- und Endprodukte als bifunktionelle und amphipolare Verbindungen häufig die Bildung von Emulsionen fördern. Bei alkalischem pH-Wert des Mediums tritt zusätzlich leicht eine Rückverseifung der gebildeten Ester ein.

3. Eine destillative fraktionierende Aufarbeitung scheidet in den meisten Fällen wegen zu eng beieinander liegender Siedepunkte von Haupt- und Nebenprodukten und wegen der relativ großen Temperaturempfindlichkeit der phenolgruppenhaltigen Carbonsäureester aus.

Nach dem derzeitigen Stand der Technik versucht man die geschilderten Probleme bei der Veresterungsreaktion phenolgruppenhaltiger Carbonsäuren dadurch zu umgehen, daß man die Umsetzung der Säure mit den jeweiligen Alkoholen unter milden Reaktionsbedingungen mehrstufig durchführt; in der letzten Stufe wird dabei zur Vervollständigung des Umsatzes der Alkohl meist in wasserfreier Form eingesetzt. Dieses Verfahren ist jedoch kompliziert, zeit- und energieaufwendig und erfordert bei Durchführung in technischem Maßstab zusätzlich den Betrieb einer Alkohol-Absolutierungsanlage.

Aus den vorstehenden Ausführungen geht somit als technische Aufgabe hervor, Bedingungen zu ermitteln, unter denen phenolgruppenhaltige Carbonsäuren in einstufiger Reaktion in ihre Niederalkylester überführt werden können. Die Umsetzung soll dabei derart vollständig und selektiv verlaufen, daß ein Anfall der obengenannten Nebenprodukte weitgehend vermieden wird und damit auf nachträgliche Aufarbeitungs- und Reinigungsoperationen verzichtet werden kann.

Erfindungsgemäß wird diese Aufgabe daduch gelöst, daß man zu einer Lösung oder Suspension der phenolgruppenhaltigen Carbonsäure und des Katalysators in einem mit Wasser nicht mischbaren Lösungsmittel einen Überschuß eines wasserhaltigen ($C_1$–$C_3$)-Alkohols in der Siedehitze kontinuierlich zudosiert und gleichzeitig über einen Wasserabscheider ein ternäres Gemisch Lösungsmittel/Alkohol/Wasser abdestilliert, wobei

(a) die Dosiergeschwindigkeit so gewählt wird, daß sich in der Vorlage eine wäßrige Unterphase abscheidet und

(b) der Wassergehalt des Alkohols so bemessen wird, daß er zu keinem Zeitpunkt der Reaktion die Grenzen von 0,1% nach unten und 1% nach oben (bezogen auf den Inhalt des Reaktionsgefäßes) überschreitet.

Die Lösung der gestellten Aufgabe – als Gegenstand der vorliegenden Erfindung – besteht demnach in der Kombination zweier Maßnahmen:

1. Die kontinuierliche Zugabe geringer Mengen Wasser, durch die die unter den drastischen Reaktionsbedingungen normalerweise eintretenden Nebenreaktionen wie Verätherung der freien Phenolgruppen und Polykondensation der phenolgruppenhaltigen Carbonsäuren überraschender Weise verhindert wird;

2. Die rasche kontinuierliche Entfernung des bei der Reaktion freigesetzten Wassers, die in Gegenwart der unbegrenzt mit Wasser mischbaren und lösungsvermittelnd wirkenden ($C_1$–$C_3$)-Alkohole nur gelingt, wenn man dafür sorgt, daß bestimmte Alkoholkonzentrationen im Reaktionsgemisch nicht überschritten werden (z. B. ca. 15 Gew.-% für Ethanol bei Verwendung von Xylol als Lösungsmittel), so daß sich in der Vorlage das ausgekreiste Wasser als untere Phase abscheidet und ein Rücklauf von Wasser ins Reaktionsgefäß

vermieden wird. Die einfachste Methode, dieses zu erreichen, besteht in der Anwendung des Teilfließbetriebs, indem der Alkohl entsprechend seinem Verbrauch durch die Veresterungsreaktion kontinuierlich nachdosiert wird. Beim Auftreten zu hoher Alkoholkonzentrationen im Reaktionsgemisch, verursacht durch zu schnelle Dosierung oder zu niedrige Reaktionsgeschwindigkeit, wird die zur Wasserausschleusung erforderliche Ausbildung zweier Phasen im Wasserabscheider verhindert, so daß Wasser ins Reaktionsgefäß zurückläuft. Die Reaktion läuft damit nicht mehr im gewünschten Sinne ab.

Durch die Kombination dieser Maßnahmen wird im Reaktionsaktionsgefäß ständig eine geringe stationäre Wasserkonzentration aufrechterhalten, die bewirkt, daß zwar die unerwünschten Nebenreaktionen nicht zum Zuge kommen, die als Hauptreaktion gewünschte Veresterung aber praktisch unbehindert ablaufen kann.

Charakteristisch für das erfindungsgemäße Verfahren ist die relativ hohe Reaktionstemperatur, die dadurch ermöglicht wird, daß weder die Alkoholkomponente noch das Wasser in größerer Konzentration im Reaktionsgemisch anwesend sind. Die hohe Temperatur ist dafür verantwortlich, daß die Umsetzung deutlich schneller abläuft, als es bei Veresterungen allgemein üblich ist. Reaktionszeiten von 1–2 Stunden sind in der Mehrzahl der Fälle ausreichend. Die Ausbeuten liegen über 90%, vielfach sogar über 95% der Theorie.

Die zur Aufrechterhaltung der stationären Wasserkonzentration im Reaktor benötigte Wassermenge wird üblicherweise in Mischung mit dem zur Veresterung eingesetzten Alkohol zudosiert. Das optimale Alkohol-Wasser-Verhältnis kann für die verschiedenen Ausgangsstoffe und Lösungsmittel mittels einfacher Vorversuche bestimmt werden. Dabei ist der Wassergehalt der Alkohole in weiten Grenzen variabel (etwa 3–25%). Somit sind auch Alkohole einsetzbar, die beispielsweise durch Aufarbeitung aus wäßrigen Lösungen gewonnen wurden. Ein weiterer Vorteil des Verfahrens besteht somit darin, daß die Verwendung wasserfreier Alkohole nicht erforderlich ist.

Bei dem erfindungsgemäßen Verfahren fallen die Veresterungsprodukte im allgemeinen in vorzüglicher Reinheit an. Restgehalte an freien Säuren unter 2% und Verunreinigungen durch Phenolether unter 1% werden regelmäßig erreicht.

Die Veresterungsreaktion kann mit besonderem Vorteil auch kontinuierlich durchgeführt werden. Die Verweilzeiten im Reaktor liegen hierbei in den meisten Fällen unter einer Stunde.

Das beschriebene Verfahren stellt somit eine einfache, rasche und wirtschaftliche Methode zur Herstellung phenolgruppenhaltiger Carbonsäureester dar. Die auf Grund der selektiven Reaktionsführung erhaltenen Rohprodukte sind in den meisten Fällen ohne weitere Reinigung weiterverwendbar.

Als Ausgangsstoffe für das erfindungsgemäße Verfahren kommen Carbonsäuren der Formel I in Betracht, die im aromatischen Molekülteil eine ortho-, meta- oder para-ständige Hydroxygruppe enthalten.

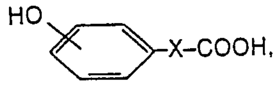

In der Formel bezeichnet X eine einfache chemische Bindung oder eine geradkettige oder verzweigte aliphatische gesättigte oder einfach oder doppelt ungesättigte Gruppierung mit vorzugsweise 1–4 C-Atomen, die direkt oder über ein Sauerstoff- oder Schwefelatom an den Kern gebunden ist.

Beispiele für einige gebräuchliche phenolgruppenhaltige Carbonsäuren sind:

2-, 3- oder 4-Hydroxy-benzoesäure
2-, 3- oder 4-Hydroxy-phenylessigsäure
2-, 3- oder 4-Hydroxy-phenylbuttersäure
2-(2-Hydroxy-phenoxy)-propionsäure
2-(3-Hydroxy-phenoxy)-propionsäure
2-(4-Hydroxy-phenoxy)-propionsäure
3-(4-Hydroxy-phenoxy)-propionsäure
2-(4-Hydroxy-phenoxy)-buttersäure
4-(4-Hydroxy-phenoxy)-buttersäure
2-, 3- oder 4-Hydroxyzimtsäure

Sofern der aliphatische Teil des Moleküls chirale Zentren enthält, versteht es sich, daß alle existierenden Stereoisomeren oder deren Mischungen in gleicher Weise der erfindungsgemäßen Veresterungsreaktion unterworfen werden können, ohne daß es zu einer Racemisierung kommt.

Als technisch wichtige Beispiele für die einzusetzenden, mit Wasser nicht mischbaren, inerten Lösungsmittel seien genannt: Vorzugsweise Aromaten mit Siedepunkten zwischen 80 und 145° wie Benzol, Toluol; Xylol (auch als technische Isomerengemisch), daneben chlorierte Aromaten wie Chlorbenzol, Halogenkohlenwasserstoffe wie Chloroform und Trichlorethylen und ($C_5$–$C_8$)-Paraffine oder deren Gemische.

Als Katalysatoren sind schwerflüchtige Mineralsäuren wie Schwefel- oder Phosphorsäure bevorzugt; auch sulfierte Aromaten (z. B. Toluolsulfosäure) oder Ionenaustauscherharze können eingesetzt werden. Flüchtige Säuren, z. B. Chlorwasserstoff, die im Prinzip ebenfalls als Katalysatoren verwendbar sind, werden unter den praktischen Bedingungen oft zusammen mit dem Reaktionswasser ausgetragen und besitzen damit nur eingeschränkte Wirksamkeit.

Die hergestellten Ester phenolgruppenhaltiger Carbonsäuren finden als bifunktionale Verbindungen mit freier Phenolgruppe in der präparativen organischen Chemie weites Interesse. Sie sind zudem wichtige Vor-, Zwischen- und Endprodukte im Bereich der pharmazeutischen Chemie, der Pflanzenschutz-Chemie, der Farbstoff-Chemie, der Textilhilfsmittel, Emulgatoren und anderer oberflächenaktiver Substanzen.

Nachstehend sind zur näheren Erläuterung einige Beispiele mit entsprechenden Gegenbeispielen aufgeführt, aus denen die Vorteile der neuen Veresterungsmethode hervorgehen.

Beispiel 1

Eine Mischung aus 7,6 g 4-Hydroxy-phenylessigsäure und 50 ml Xylol wird in einem mit Rückflußkühler und Wasserabscheider ausgerüsteten Kolben mit 0,5 ml konz. Schwefelsäure versetzt und unter Rühren auf 120 °C erhitzt. Innerhalb von 80 min wird eine Mischung aus 30 ml Xylol, 52 g n-Propanol und 5,2 g Wasser tropfenweise zugegeben und gleichzeitig über den Wasserabscheider aus der unter Rückfluß siedenden Lösung 74 g Destillat abgenommen. Aus diesem Lösungsmittelgemisch scheiden sich 1,7 g einer wäßrigen Unterphase ab. Die gaschromatographische Analyse der Reaktionsmischung nach Beendigung der Zugabe ergibt folgende Werte (ohne Lösungsmittelanteile):

| | |
|---|---|
| 4-Hydroxy-phenylessigsäure-n-propylester | 97,8% |
| 4-n-Propyloxy-phenylessigsäure-n-propylester | 0,4% |
| 4-Hydroxy-phenylessigsäure | 1 % |

Zur Aufarbeitung wäscht man das Reaktionsgemisch mit Wasser oder einer verdünnten wäßrigen Natriumhydrogencarbonatlösung und dampft das organische Lösungsmittel unter vermindertem Druck ab. Die Ausbeuten an Ester liegen in praktisch allen Fällen über 90–95% d. Th. Das Produkt ist für viele Verwendungen ohne weitere Reinigung brauchbar.

Vergleichsbeispiel 1

Unter gleichen Bedingungen wie in Beispiel 1 werden 7,6 g 4-Hydroxy-phenylessigsäure mit n-Propanol verestert. Im Unterschied zu Beispiel 1 wird bei diesem Versuch kein Wasser zugegeben. Als Destillat erhält man in diesem Falle 67 g Lösungsmittelgemisch, aus denen sich 0,8 g wäßrige Unterphase abscheiden. Die gaschromatographische Analyse (ohne Lösungsmittelanteile) ergibt für das Reaktionsgemisch nach Beendigung der Xylol/n-Propanolzugabe:

| | |
|---|---|
| 4-Hydroxy-phenylessigsäure-n-propylester | 89,6% |
| 4-n-Propyloxy-phenylessigsäure-n-propylester | 1,5% |
| 4-Hydroxy-phenylessigsäure | 1 % |

Beispiel 2

27,2 g 4-Hydroxy-benzoesäure werden in 200 ml Xylol suspendiert und mit 2 ml konz. Schwefelsäure versetzt. Man heizt die Mischung auf 125 °C auf und dosiert anschließend innerhalb von 90 min insgesamt 43 g 90%iges wäßriges Ethanol zu. Gleichzeitig destilliert man über den Wasserabscheider 40 g Lösungsmittelgemisch ab. Die Reaktionstemperatur sinkt vorübergehend auf ca. 120 °C ab. Durch weiteres Rückflußkochen erreicht man ein langsames Wiederansteigen der Temperatur auf 123 °C. Nach 14 Stunden wird eine gaschromatographische Analyse durchgeführt. Befund (ohne Lösungsmittelanteile):

| | |
|---|---|
| 4-Hydroxy-benzoesäure-ethylester | 93,2% |
| 4-Hydroxy-benzoesäure | 2,0% |

4-Ethoxy-benzoesäure-ethylester war nicht nachzuweisen. Die Aufarbeitung des Reaktionsansatzes kann nach konventionellen Methoden erfolgen. Die Ausbeute beträgt 90% d. Th.

Beispiel 3

In einem mit Rückflußkühler und Wasserabscheider versehenen Rührkolben wird eine Suspension von 9,1 g 2-(4-Hydroxy-phenoxy)-propionsäure in 50 ml Xylol vorgelegt. Nach Zugabe von 0,5 ml konz. Schwefelsäure heizt man die Mischung auf 125 °C und tropft dann bei dieser Temperatur in 50 min 44,0 g wäßriges Ethanol (Wassergehalt 9 Gew.-%) zu. Das während dieser Zeit abdestillierende Lösungsmittelgemisch wird über den Wasserabscheider ausgetragen. Es fallen insgesamt 67 g Destillat (Xylol/Ethanol-Wasser-Gemisch) an, aus denen sich eine wäßrige Unterphase von 2,0 g abscheidet. Um die Flüssigkeitsmenge im Reaktionskolben konstant zu halten, werden im Verlauf der Umsetzung weitere 25 ml Xylol nachdosiert. Die gaschromatographische Analyse der destillierbaren Anteile des Rohprodukts (ohne Lösungsmittel) liefert folgende Werte:

| | |
|---|---|
| 2-(4-Hydroxy-phenoxy)-propionsäure-ethylester | 92,18% |
| 2-(4-Ethoxy-phenoxy)-propionsäure-ethylester | 1,79% |
| 2-(4-Hydroxy-phenoxy)-propionsäure | 1,57% |

Vergleichsbeispiel 3

Unter analogen Bedingungen wie in Beispeil 3 werden 9,1 g 2-(4-Hydroxy-phenoxy)-propionsäure in 50 ml Xylol in Gegenwart von 0,5 ml konz. Schwefelsäure durch Zugabe von 40,0 g wasserfreiem Ethanol verestert. Wiederum werden zum Volumenausgleich 25 ml Xylol nachdosiert. Bei diesem Versuch fallen in 50 min 60 g Lösungsmitteldestillat an, aus denen sich 1,4 g wäßrige Unterphase abscheiden.

Gaschromatographische Analyse der destillierbaren Anteile des Rohprodukts (ohne Lösungsmittel):

| | |
|---|---|
| 2-(4-Hydroxy-phenoxy)-propionsäure-ethylester | 91,95% |
| 2-(4-Ethoxy-phenoxy)-propionsäure-ethylester | 4,53% |
| 2-(4-Hydroxy-phenoxy)-propionsäure | $<0,1$ % |

Die Aufarbeitung der Rohproduktlösungen erfolgt bei beiden Versuchen in gleicher Weise nach bekannten Verfahren: nach Entfernung der Schwefelsäure durch Ausschütteln der xylolischen Lösung mit Wasser oder einer verdünnten Lösung von Natriumhydrogencarbonat in Wasser wird das organische Lösungsmittel durch Abdestillieren unter vermindertem Druck abgezogen. Die Ausbeuten an Veresterungsprodukt liegen bei 95% d. Th.

Beispiel 4

Chargenverfahren – optimierte Bedingungen (Apparatur wie in Beispiel 3).

Die Suspension von 9,1 g 2-(4-Hydroxy-phenoxy)-propionsäure in 50 ml Xylol wird nach Zusatz von 0,5 ml konz. Schwefelsäure auf 120 °C geheizt. Der auf der Apparatur angebrachte Wasserabscheider ist von einem vorhergehenden Versuch mit einer Mischung aus Xylol und Ethanol im Verhältnis 5,67 : 1 gefüllt. Innerhalb von 60 min tropft man zur Reaktionsmischung insgesamt 22,0 g

91 gew.-%iges wäßriges Ethanol hinzu. Nach ca. 10 min Reaktionsdauer ist eine klare Lösung entstanden. Das übergehende Lösungsmitteldestillat fließt über den Wasserabscheider in den Reaktionskolben zurück. Im Wasserabscheider trennen sich während der Reaktionszeit 4,4 g wäßrige Unterphase ab; diese werden nicht wieder in den Reaktor zurückgeführt. Die gaschromatographische Analyse der destillierbaren Anteile nach Beendigung der Ethanolzugabe liefert folgende Werte (ohne Lösungsmittelanteil):

| | |
|---|---|
| 2-(4-Hydroxy-phenoxy)-propionsäure-ethylester | 96,51% |
| 2-(4-Ethoxy-phenoxy)-propionsäure-ethylester | 0,48% |
| 2-(4-Hydroxy-phenoxy)-propionsäure | 0,45% |

Die Ausbeute an isoliertem Rohmaterial nach Auswaschen der Schwefelsäure und Abdampfen des Lösungsmittels liegt über 100%; bei Berücksichtigung der ermittelten Estergehalte ergeben sich in Reihenversuchen Ausbeuten zwischen 95 und 98% d. Th.

Beispiel 5

Kontinuierliches Verfahren.

In ein 1 l-Reaktionsgefäß, das mit Rückflußkühler, Wasserabscheider und einem Überlauf ausgerüstet ist, werden kontinuierlich eindosiert:

246 g/h einer ca. 70 °C warmen 35 Gew.-%igen Lösung von 2-(4-Hydroxy-phenoxy)-propionsäure in einem Xylol-Ethanol-Gemisch mit einem Ethanol-Anteil von 8 Gew.-%. Die organische Säure liegt als technisches Rohprodukt mit einer Anzahl von Begleitprodukten vor;

270 g/h Xylol (technisches Isomerengemisch);

4 g/h 96%ige Schwefelsäure;

40 g/h 95,8%iges wäßriges Ethanol.

Unter Rühren wird diese Mischung auf 125 °C erhitzt, dabei tritt kräftiges Sieden ein. Die sich im Wasserabscheider als Unterphase abtrennende wasserhaltige Phase wird kontinuierlich abgezogen, die Oberphase läuft ständig in das Reaktionsgefäß zurück. Der am Reaktor befindliche Überlauf wird so eingeregelt, daß im Kolben eine Standhaltung von ca. 600 ml erfolgt. Nachdem sich ein stationärer Zustand eingestellt hat, können am Wasserabscheider 40 g/h der Unterphase mit einer Zusammensetzung: 6 Teile Ethanol, 3 Teile Wasser, 1 Teil Xylol entnommen werden. Gleichzeitig fallen am Überlauf des Reaktors

520 g/h einer xylolischen Lösung mit einem Gehalt von durchschnittlich 19,5 Gew.-% 2-(4-Hydroxy-phenoxy)-propionsäure-ethylester an; das entspricht einer Ausbeute von 97% d. Th.

Bei dem im Beispiel gewählten Durchsatz beträgt die Verweilzeit etwa eine Stunde. Mit praktisch gleichem Erfolg sind Verweilzeiten bis herab zu ca. 30 min möglich. In einem Testlauf wurde der beschriebene Versuch über 40 Stunden kontinuierlich durchgeführt. Vier in regelmäßigen Abständen gezogene Analysenproben ergaben folgende Befunde für die charakteristischen Nebenprodukte (in Gewichtsteilen jeweils bezogen auf 100 Gewichtsteile 2-(4-Hydroxy-phenoxy)-propionsäure-ethylester):

2-(4-Ethoxy-phenoxy)-propionsäure-ethylester
0,14; 0,33; 0,13; 0,31

2-(4-Hydroxy-phenoxy)-propionsäure
1,73; 1,96; 1,33; 1,98

Die Aufarbeitung erfolgt nach konventionellen Verfahren.

Alle Prozentangaben bedeuten Gewichtsprozent.

## Patentansprüche

1. Verfahren zur Veresterung phenolgruppenhaltiger Carbonsäuren mittels ($C_1$–$C_3$)-Alkoholen in Gegenwart saurer Katalysatoren ohne gleichzeitige Verätherung der phenolischen Hydroxylgruppen, dadurch gekennzeichnet, daß man zu einer Lösung oder Suspension der phenolgruppenhaltigen Carbonsäure der Formel

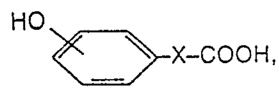

in der X eine einfache chemische Bindung oder eine geradkettige oder verzweigte aliphatische gesättigte oder einfach oder doppelt ungesättigte Gruppierung mit vorzugsweise 1–4 C-Atomen, die direkt oder über ein Sauerstoff- oder Schwefelatom an den Kern gebunden ist, bedeutet, und des Katalysators in einem mit Wasser nicht mischbaren Lösungsmittel einen Überschuss eines wasserhaltigen ($C_1$–$C_3$)-Alkohols in der Siedehitze kontinuierlich zudosiert und gleichzeitig über einen Wasserabscheider ein ternäres Gemisch Lösungsmittel/Alkohol/Wasser abdestilliert, wobei

(a) die Dosiergeschwindigkeit so gewählt wird, daß sich in der Vorlage eine wäßrige Unterphase abscheidet, und

(b) der Wassergehalt des Alkohols so bemessen wird, daß er zu keinem Zeitpunkt der Reaktion die Grenzen von 0,1 Gew.-% nach unten und 1 Gew.-% nach oben (bezogen auf den Inhalt des Reaktionsgefäßes) überschreitet.

2. Verfahren nach Anspruch 1, dadurch gekennzeichnet, daß man den durch Abdestillieren des Lösungsmittels entstehenden Lösungsmittelverlust während der Reaktion durch Zugabe von Lösungsmittel ausgleicht.

3. Verfahren nach Ansprüchen 1 oder 2, dadurch gekennzeichnet, daß man als Lösungsmittel einen aromatischen Kohlenwasserstoff mit einem Siedepunkt von 80–145 °C verwendet.

4. Verfahren nach Anspruch 3, dadurch gekennzeichnet, daß man als Lösungsmittel Xylol verwendet.

5. Verfahren nach einem oder mehreren der Ansprüche 1 bis 4, dadurch gekennzeichnet, daß die Carbonsäure 4-Hydroxy-phenylessigsäure, 4-Hydroxybenzoesäure oder 2-(4-Hydroxy-phenoxy)-propionsäure ist.

**Claims**

1. A process for esterifying a carboxylic acid which contains a phenol group by means of a ($C_1$–$C_3$)-alcohol in the presence of an acid catalyst without simultaneous etherification of the phenolic hydroxyl group, which comprises continuously metering into a boiling hot solution or suspension of the carboxylic acid which contains a phenol group and has the formula

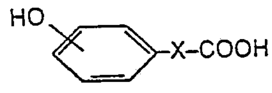

where X is a single chemical bond or a straight-chain or branched aliphatic saturated, monounsaturated or diunsaturated grouping of preferably 1–4 carbon atoms which is bonded direct or via an oxygen or sulfur atom to the nucleus, and of the catalyst in a water-immiscible solvent, an excess of a water-containing ($C_1$–$C_3$)-alcohol and simultaneously distilling off, via a water separator, a ternary solvent/alcohol/water mixture, wherein

(a) the metering rate is such that an aqueous bottom phase separates out in the receiving flask and

(b) the water content of the alcohol is so apportioned as not to breach a lower limit of 0.1% by weight or an upper limit of 1% by weight (based on the contents of the reaction vessel) at any time in the course of the reaction.

2. The process as claimed in claim 1, wherein the solvent loss caused by distilling off the solvent is made up during the reaction by the addition of solvent.

3. The process as claimed in claims 1 or 2, wherein the solvent is an aromatic hydrocarbon having a boiling point of 80–145 °C.

4. The process as claimed in claim 3, wherein the solvent is xylene.

5. The process as claimed in one or more of claims 1 to 4, wherein the carboxylic acid is 4-hydroxyphenylacetic acid, 4-hydroxybenzoic acid or 2-(4-hydroxyphenoxy)propionic acid.

**Revendications**

1. Procédé d'estérification d'acides carboxyliques à groupes phénoliques avec des alcools en $C_1$ à $C_3$ en présence de catalyseurs acides, sans éthérification des hydroxyles phénoliques, procédé caractérisé en ce que l'on ajoute progressivement à une solution ou à une suspension d'un acide carboxylique de formule:

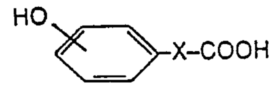

(dans laquelle X représente une liaison chimique simple ou un groupe aliphatique à chaîne linéaire ou ramifiée, saturé ou avec une ou deux insaturations et qui a de préférence de 1 à 4 atomes de carbone, lié au cycle directement ou par l'intermédiaire d'un atome d'oxygène ou de soufre) et du catalyseur dans un solvant non-miscible à l'eau, un excès d'un alcool aqueux en $C_1$ à $C_3$, en continu et à la température d'ébullition, et en même temps on élimine par distillation un mélange ternaire du solvant, de l'alcool et de l'eau, en faisant passer ce mélange par un séparateur d'eau, et ceci en opérant:

(a) à une vitesse d'addition telle qu'il se sépare dans l'appareil une phase inférieure aqueuse, et

(b) avec une teneur en eau de l'alcool qui a tout moment de la réaction reste dans les limites de 0,1 à 1% par rapport au contenu du récipient de réaction, c'est-à-dire du mélange réactionnel.

2. Procédé selon la revendication 1, caractérisé en ce que l'on compense la perte de solvant par distillation au cours de la réaction en ajoutant une nouvelle quantité de solvant.

3. Procédé selon la revendication 1 ou 2, caractérisé en ce que le solvant est un hydrocarbure aromatique ayant un point d'ébullition de 80 à 145 °C.

4. Procédé selon la revendication 3, caractérisé en ce que le solvant est du xylène.

5. Procédé selon une ou plusieurs des revendications 1 à 4, caractérisé en ce que l'acide carboxylique est l'acide 4-hydroxyphénylacétique, l'acide 4-hydroxybenzoïque ou l'acide 2-(4-hydroxyphénoxy)-propionique.